## Europäisches Patentamt

## European Patent Office

## Office européen des brevets

(11) Veröffentlichungsnummer: **0 295 597**
**A2**

(12) **EUROPÄISCHE PATENTANMELDUNG**

(21) Anmeldenummer: 88109349.6

(22) Anmeldetag: 11.06.88

(51) Int. Cl.4: **C07K 13/00 , C12N 15/00 , C12P 21/02 , A61K 37/02 , G01N 33/53 , //A61K35/16**

Patentansprüche für folgende Vertragsstaaten:
ES + GR.

(30) Priorität: 19.06.87 DE 3720246

(43) Veröffentlichungstag der Anmeldung:
21.12.88 Patentblatt 88/51

(84) Benannte Vertragsstaaten:
AT BE CH DE ES FR GB GR IT LI LU NL SE

(71) Anmelder: **BEHRINGWERKE Aktiengesellschaft**
**Postfach 1140**
**D-3550 Marburg 1(DE)**

(72) Erfinder: **Langner, Klaus-Dieter, Dr.**
**Lindenweg 14**
**D-3550 Marburg(DE)**
Erfinder: **Egon Amann, Dr.**
**Sachsenring 8**
**D-3550 Marburg(DE)**
Erfinder: **Küpper, Hans, Dr.**
**Biegenstrasse 39**
**D-3550 Marburg(DE)**
Erfinder: **Zettlmeissl, Gerd, Dr.**
**Am Hofacker 15**
**D-3551 Lahntal(DE)**
Erfinder: **Huber, Bernard, Dr.**
**Kolbergerstrasse 19**
**D-8000 München 80(DE)**

(74) Vertreter: **Meyer-Dulheuer, Karl-Hermann, Dr. et al**
**HOECHST Aktiengesellschaft Zentrale Patentabteilung Postfach 80 03 20**
**D-6230 Frankfurt/Main 80(DE)**

(54) **Faktor VIII:C-ähnliches Molekül mit Koagulationsaktivität.**

(57) Die Erfindung betrifft eine Mutante von Faktor VIII : C, wobei dem neuen Molekül die Aminosäuren $Ser^{741}$ bis $Arg^{1689}$ fehlen. Dadurch wird von den bisher als essentiell angesehenen 4 Thrombinspaltstellen nur die Spaltstelle bei $Arg^{372}$ beibehalten. Die Spaltstelle bei $Arg^{1648}$ fehlt, von den Spaltstellen bei $Arg^{740}$ und $Arg^{1689}$ sind die angrenzenden Aminosäuren vorhanden, durch deren Verknüpfung eine neue Spaltstelle entsteht. Das neue Protein besitzt neben einer dem authentischen Protein sehr ähnlichen Prokoagulationsaktivität und biologischen Halbwertszeit eine schnellere Aktivierung durch Thrombin.

FIG. 17

## Faktor VIII:C-ähnliches Molekül mit Koagulationsaktivität

Die Erfindung betrifft eine Mutante von Faktor VIII:C (F VIII:C), die dadurch gekennzeichnet ist, daß dem neuen Molekül die Aminosäuren $Ser^{741}$ bis $Arg^{1689}$ fehlen, wobei sich die Numerierung der Aminosäuren auf das reife F VIII:C-Molekül (nach Abspaltung das Signalpeptids) bezieht. Letzteres Molekül besteht aus 2332 Aminosäuren ($Ala^1$ -$Tyr^{2332}$). Das neue Protein besitzt trotz drastischer Veränderungen bezüglich struktureller Parameter (z.B. Fehlen von mehreren SH-Gruppen und Glykosylierungsstellen) und bezüglich der Anzahl der Thrombinspaltstellen eine dem authentischen Protein sehr ähnliche Prokoagulationsaktivität.

In der Kaskade der Blutgerinnung nimmt F VIII:C eine zentrale Stellung ein, wobei er als Kofaktor in der Aktivierung von Faktor X durch aktivierten Faktor IX dient. F VIII:C stellt das Protein dar, das bei Menschen, die an klassischer Haemophilie A erkrankt sind, defekt oder gar nicht vorhanden ist. Haemophilie A ist eine Krankheit, die X-chromosal vererbt wird und bei männlichen Individuen mit einer Häufigkeit von etwa 0,01 % auftritt.

Sowohl für den menschlichen F VIII:C kodierende cDNA als auch das komplette F VIII:C-Gen wurden isoliert und charakterisiert (Gitschier et al., 1984, Nature 312, 326-330; Wood et al. 1984, Nature 312, 330-337; Vehar et al., 1984, Nature 312, 337-342; Toole et al., 1984, Nature 312, 342-347). Die cDNA kodiert für 2332 Aminosäuren des reifen Proteins, was einem Molekulargewicht von 264 763 d entspricht. Die Reinigung von F VIII:C aus kommerziellen F VIII:C-Konzentraten führte zu einer Reihe von mit biologischer Aktivität assoziierten Polypeptiden mit Molekulargewichten von 80 000- 200 000 d, wobei eine 90 000 d und eine 80 000 d umfassende Proteinkette eine wichtige Zwischenstufe bei der Aktivierung darstellen (Andersson et al., 1986, Proc. Natl. Acad. Sci., USA 83, 2979-2983). Im intakten Molekül sind diese beiden Polypeptidketten durch eine $Ca^{2+}$-Brücke verbunden und stellen in dieser Form eine durch Thrombin prozessierte, partiell aktive Form von F VIII:C dar.

Die 90 000 d N-terminal liegende Kette entsteht aus F VIII:C-Vorläufermolekülen durch Thrombinspaltung an der Position $Arg^{740}$ ($Ala^1$-$Arg^{740}$), wogegen das 80 000 d-Protein durch Thrombinspaltung an der Position $Arg^{1648}$ ($Glu^{1649}$-$Tyr^{2332}$) zustande kommt. Zum Erreichen der vollen Aktivierung von F VIII:C wird das N-terminal liegende 90 000 d-Fragment an Position $Arg^{372}$ (entstehende Fragmente: 50 000 d und 40 000 d) und das C-terminale 80 000 d-Fragment an Position $Arg^{1689}$ (entstehende Fragmente 73 000 d und 7 000 d) durch Thrombin gespalten (Eaton et al., 1986, Biochemistry 25, 505-512; Eaton et al., 1986 Biochemistry 25, 8343-8347; Andersson et al., s.o.).

Basierend auf einer modifizierten F VIII:C-cDNA konnten von Toole et al. (1986, Proc. Natl. Acad. Sci., USA 83, 5939-5942) biologisch voll aktive Proteinmoleküle in animalen Zellen exprimiert werden, in denen der Bereich zwischen den Aminosäuren $Ser^{982}$ und $Leu^{1562}$ bzw. zwischen den Aminosäuren $Thr^{760}$ und $Asn^{1639}$ deletiert ist. Diese Moleküle tragen jedoch alle vier für die Aktivierung als essentiell angesehenen Thrombinspaltstellen (Positionen $Arg^{372}$, $Arg^{740}$, $Arg^{1648}$, $Arg^{1689}$). Bei diesen Experimenten wurden die deletierten Proteine als eine durchgehende Kette exprimiert. Burke et al. (1986, J. Biol. Chem. 261, 12574-12578) exprimierten die Bereiche $Ala^1$-$Arg^{740}$ und $Glu^{1649}$-$Tyr^{2332}$ als separate Ketten zusammen in einer Wirtszelle und konnten im Medium ebenfalls biologisch aktive F VIII:C-Moleküle, die die beiden Ketten in assoziierter Form enthalten, nachweisen. Die beiden Proteinketten entsprechen den bei F VIII:C aus Plasma durch Thrombinspaltung aus den Stellen $Arg^{740}$ bzw. $Arg^{1648}$ entstehenden Spezies mit einem Molekulargewicht von 90 000 d bzw. 80 000 d. Wie bei F VIII:C aus Plasma sind in diesen Spezies die zur weiteren Aktivierung nötigen Thrombinspaltstellen an den Positionen $Arg^{372}$ und $Arg^{1689}$ vorhanden.

Bei allen bisher beschriebenen Mutanten des F VIII:C-Moleküls sind somit die beiden zuletzt erwähnten Thrombinspaltstellen in ihrer natürlichen Form vorhanden.

Durch Deletion der Aminosäuren $Ser^{741}$ bis $Arg^{1689}$ in der Mutante, die Gegenstand dieser Erfindung ist, wird von den vier für die Aktivierung von natürlichem F VIII:C als essentiell angesehenen Thrombinspalt-stellen (an den Positionen $Arg^{372}$, $Arg^{740}$, $Arg^{1648}$ und $Arg^{1689}$) nur die Spaltstelle bei $Arg^{372}$ beibehalten. Die Spaltstelle bei $Arg^{1648}$ fehlt vollkommen, von den Spaltstellen bei $Arg^{740}$ und $Arg^{1689}$ sind nur die angrenzenden Aminosäuren vorhanden. Durch die angegebene Deletion werden die Aminosäuren $Arg^{740}$ und $Ser^{1690}$ durch eine Peptidbindung verknüpft. Diese Verknüpfung stellt eine neue Thrombinspaltstelle in diesem neuen, mutierten von F VIII:C abgeleiteten und als durchgehende Kette exprimierten Molekül dar.

Das für dieses neue Protein kodierende neuartige F VIII:C-Gen konnte in Animalzellen eingeschleust und dort zur Expression gebracht werden. In dem Kultur-Überstand von derartig veränderten Animalzellen konnte ein Molekül mit F VIII:C-spezifischer Prokoagulationsaktivität nachgewiesen werden. Durch Thrombinbehandlung kann die Aktivität dieses Proteins um eine Größenordnung gesteigert werden.

Die Aktivierung der erfindungsgemäßen Mutante durch Thrombin führt aufgrund der Entfernung potentieller Aktivierungsspaltstellen zu definierten, gegenüber dem Wildtypprotein von von anderen Gruppen beschriebenen Deletionsmutanten einfacheren Spaltungsmustern. Hierdurch ergibt sich eine direkte experimentelle Zugriffsmöglichkeit auf die Korrelation von Thrombinspaltung und biologischer Aktivität. Der vereinfachte Aktivierungsmechanismus kann beim Einsatz des Moleküls in der Substitutionstherapie vorteilhaft sein. Überraschenderweise hat das neue Molekül eine dem Wildtyp gegenüber zumindest gleichlange biologische Halbwertszeit bei der Inaktivierung durch Thrombin, obwohl hinsichtlich des ursprünglichen Proteins zwei Thrombinspaltstellen fehlen. Die Aktivierung des neuen Moleküls ist um mindestens den Faktor 2 schneller.

In der neuen Mutante wurden durch die Deletion des Polypeptidbereichs $Ser^{741}$-$Arg^{1689}$ 20 der 25 potentiellen Asparagin-Glykosylierungsstellen (Vehar et al., s.o.) entfernt. Diese Tatsache und das Fehlen eines großen erhablichen Proteinanteils vereinfacht die Herstellung mittels gentechnologischer Techniken, weil die hierzu verwendeten Wirtsorganismen weniger Proteinsynthese- und Glykosylierungsleistung vollbringen müssen. Es ist bekannt, daß heterologe Proteine bei der Synthese in Hefen und in animalen Zellen nicht exakt wie das entsprechende natürlich vorkommende Protein glykosyliert werden (Bröker et al. (1987), Biochim. Biophys. Acta 908, 203-213; Conradt et al., 1986, Carbohydrate Res. 149, 443-450). Diese "falsche" Glykosylierung kann zu reduzierter physiologischer Halbwertszeit und unerwünschten Immunreaktionen führen. Die Entfernung des grössten Teils der potentiellen N-Glykosylierungsstellen vermindert somit die Möglichkeit der "falschen" Glykosylierung im Wirtsorganismus drastisch.

## Beispiele

### 1. Klonierung von F VIII:C

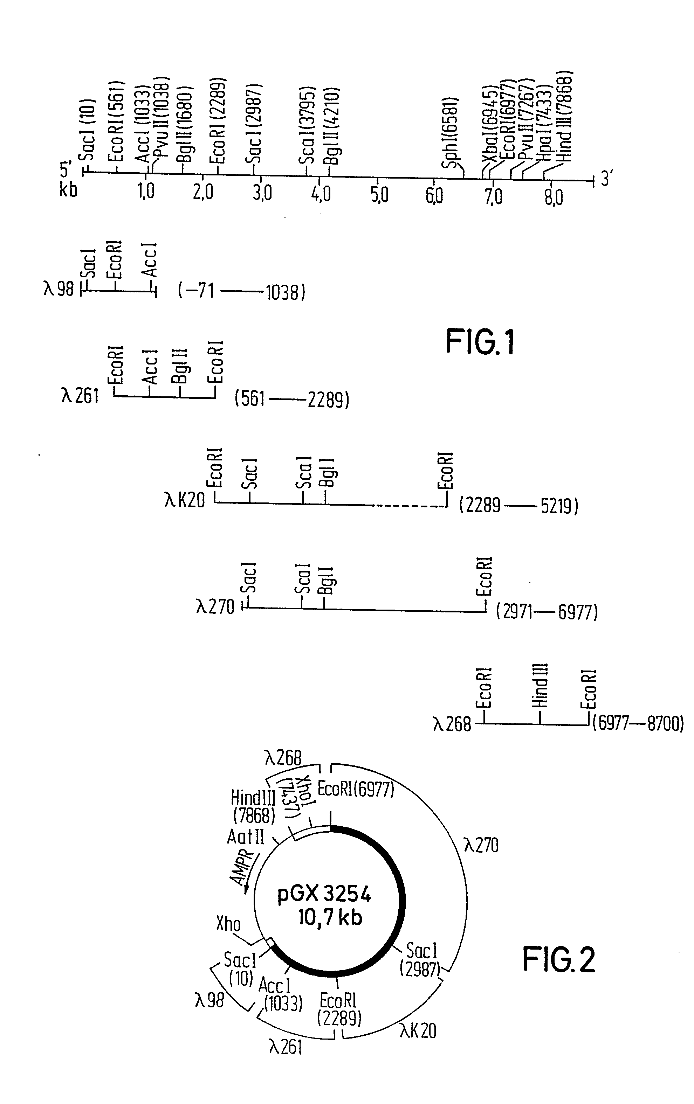

Zur Klonierung F VIII:C-spezifischer DNA-Sequenzen wurden cDNA aus humanem Lebergewebe nach bekannten Methoden hergestellt (Maniatis, T., Fritsch, E.F. und Sambrook, J. 1982, Molecular Cloning, Cold Spring Harbor Laboratory). Mit dieser cDNA wurden Genbanken in den Vektoren λgt10 und λgt11 etabliert (Young, R.A. und Davis, R.W. (1983), Proc. Natl. Acad. Sci. USA 80, 1194-1198). Weiterhin wurden mehrere humane genomische Genbanken in dem Phagenvektor λ Charon 4a (Lawn, R., et al. (1978) Cell 15, 1157-1174) hergestellt. Diese Genbanken wurden auf F VIII:C-spezifische DNA-Sequenzen hin untersucht. Grundlage zur Isolierung aller F VIII:C-spezifischen Klone waren 17 synthetische Oligonukleotide. Diese Oligonukleotide basieren auf der von Wood et al. publizierten Sequenz (Wood et al. (1984), Nature 312, 330-337) und entsprechen kurzen Teilsequenzen des kodierenden oder des nichtkodierenden Stranges (Tab. 1). Die genannten Oligonukleotide dienten entweder nach radioaktiver Endmarkierung als Sonde zum "Screening" der obengenannten Genbanken oder zum spezifischen "Priming" neuer cDNA-Synthesen mit humaner Leber-mRNA. Durch spezifische Hybridisierung wurden aus den cDNA-Banken vier verschiedene Klone und aus den genomischen Genbanken ein Klon isoliert, die zusammengenommen die gesamte F VIII:C-Sequenz abdecken (Fig. 1; Intron-DNA im genomischen DNA-Klon λK20 ist durch eine unterbrochene Linie dargestellt. Die Basennumerierung in Fig. 1 bezieht sich auf Exon-DNA). Aus diesen Klonen wurde durch Isolierung der in der Fig. 2 gezeigten Restriktionsfragmente und Ligierung dieser Fragmente in die Vektoren pUC18 oder pUC19 (Norrander et al. (1983) Gene 26, 101-106; Yanish-Perron, et al. (1985) Gene 33, 103-119) über mehrere Zwischenschritte ein Plasmid erstellt, welches die vollständige F VIII:C-Kodierungssequenz 101-106; Yanish-Perron, et al. (1985) Gene 33, 103-119) über mehrere Zwischenschritte ein Plasmid erstellt, welches die vollständige F VIII:C-Kodierungssequenz trägt. Dieses Plasmid, pGX3254 (Fig. 2) ist 10 623 Basenpaare lang und enthält die F VIII:C-Kodierungsregion von 7 056 Basenpaaren (entsprechend 2351 Aminosäuren von F VIII:C) sowie 71 Basenpaare der 5′-untranslatierten Region und 821 Basenpaare der 3′-untranslatierten Region. Die gesamte F VIII:C-Kodierungssequenz in pGX3254 wurde nach der Dideoxymethode (Sanger et al. (1977) Proc. Natl. Acad. Sci. USA 74, 5463-5467) bestimmt und ist identisch mit der von Wood et al. publizierten F VIII:C-Sequenz. Im folgenden beziehen sich alle angegebenen Sequenzkoordinaten auf die von Wood et al. publizierte F VIII:C-Sequenz. Die Vektorsequenzen von pGX3254 bestehen aus Anteilen von pUC18 und aus Anteilen von pUC19. Beim Zusammenbau der F VIII:C-Kodierungssequenz mittels geeigneter Restriktionsstellen auf Plasmidvektoren wurde ein synthetischer XhoI-Linker (GCTCGAGC) an der natürlich vorkommenden HpaI-Stelle in der 3′-nichtkodierenden Region (Position 7433) der F VIII:C-Sequenz insertiert (pGX3243). Weiterhin wurde durch Subklonierung eines PvuII-Fragments, welches die F VIII:C-Sequenz von -71 bis 1038 aus λ98 (Fig. 1) trägt, in die SmaI-Stelle des Vektors pGX3237 (Polylinkerregion: ClaI-SphI-XhoI-XbaI-EcoRI-SmaI-BamHI-SalI-PstI-HindIII) das Plasmid pGX3238 erhalten, welches eine einzelne XhoI-Stelle an der Position -104 (5′ vor dem F VIII:C-ATG-Initiationscodon) trägt. Durch Verknüpfen der 5'-F VIII:C-Regionen von pGX3238, der in Fig. 2 gezeigten Subfragmente und der 3'-F VIII:C-Regionen von pGX3243 entstand pGX3254, welches nun die gesamte F VIII:C-Sequenz, flankiert durch XhoI-Stellen, trägt. In Fig. 2 ist die kodierende Region durch - schwarz ausgefüllte Linien, die nicht kodierende Region durch dünne Konturierung bezeichnet.

## 2. Herstellen der Deletionsmutanten pGX3788 und pGX3789

Bezogen auf die natürlich vorkommende F VIII:C-cDNA Sequenz in pGX3254 wurden zwei Delationsmutanten hergestellt. Es wurde die Technik der "site-directed mutagenesis" nach Zoller und Smith (Zoller, M.J. und Smith, M. (1983) in: Methods Enzymol. 100: 468-500) benutzt. Das Plasmid pGX3788 enthält eine Sequenz für F VIII:C, in der die kodierende Information für die Aminosäuren 741-1689 (= F VIII:C Δ741-1689) deletiert wurde. Plasmid pGX3789 enthält eine Sequenz, in der die Information für die Aminosäuren 816-1598 (= F VIII:C Δ816-1598) deletiert wurde. Analog zu der zuvor beschriebenen Konstruktion von pGX3254 wurden pGX3788 und pGX3789 ebenfalls mit flankierenden XhoI-Stellen an gleicher Position hergestellt.

### a) Strategie zur Mutagenese

Es wurde vermutet, daß die repetitiven Sequenzen in der F VIII:C-cDNA Sequenz Ursache für möglicherweise auftretende Spontan-Deletionen während der Mutagenese sein könnten. Aus diesem Grunde wurde die Mutagenese an Subklonen der pGX3254-Sequenz durchgeführt, die solche repetitiven Sequenzen nicht tragen. Die Subklone wurden so ausgewählt, daß nach der erfolgten Mutagenese durch die Wahl geeigneter Restriktionsstellen ein Plasmid zusammengebaut werden konnte, das für ein F VIII:C-Molekül mit der gewünschten Deletion codiert.

### b) Herstellen von Subklonen zur Mutagenese

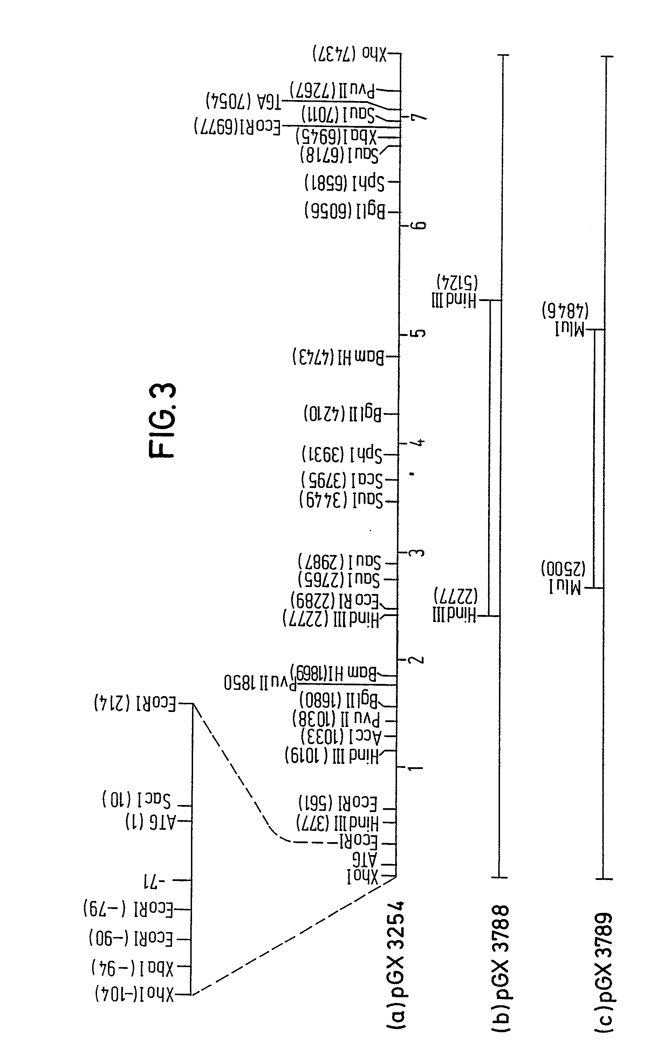

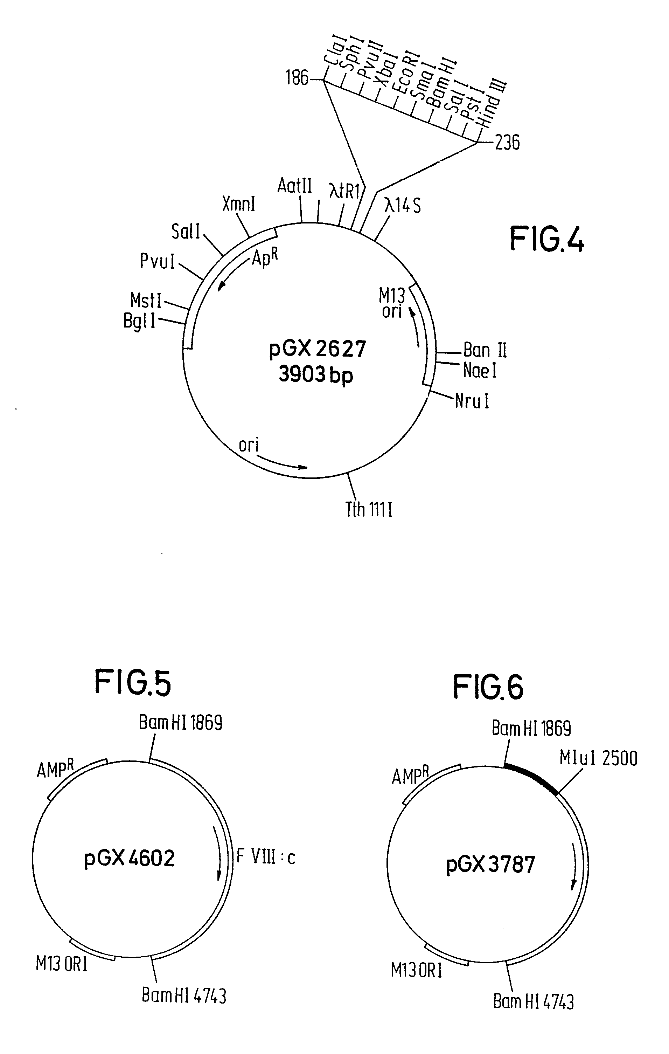

Zwei F VIII:C Fragmente wurden zur Mutagenese subkloniert: Ein 2874 bp langes BamHI-Fragment (Position 1869-4743; Fig. 3a) und ein 2650 bp langes SphI Fragment (Position 3931-6581; Fig. 3a). Der Vektor für die Subklonierung war pGX2627 (Fig. 4). Er trägt einen Polylinker mit Schnittstellen für die Restriktionsenzyme:
ClaI-SphI-PvuII-XbaI-EcoRI-SmaI-BamHI-ASalI-PstI-HindIII sowie neben einem plasmidspezifischen Replikationsorigin den des Bakteriophagen M13. Letzteres erlaubt die Verpackung von Einzelsträngen der Plasmid DNA mit Hilfe einer Superinfektion mit M13 Bakteriophagen. Vektor pGX2683 trägt die Polylinkerregion in entgegengesetzter Orientierung im Vergleich zu pGX2627. Die so erhaltene einzelsträngige DNA diente anschließend als Template für die Oligonukleotidinduzierte Mutagenese. Subklon pGX4602 (Fig. 5), trägt das obengenannte BamHI-Fragment in der BamHI-Stelle von pGX2627. Subklon pGX4604 (Fig. 7) trägt das obengenannte SphI-Fragment in der SphI-Stelle von pGX2627 und Subklon pGX4605 (Fig. 9) trägt das gleiche SphI Fragment in der entgegengesetzten Orientierung in pGX2627.

### c) Mutagenese

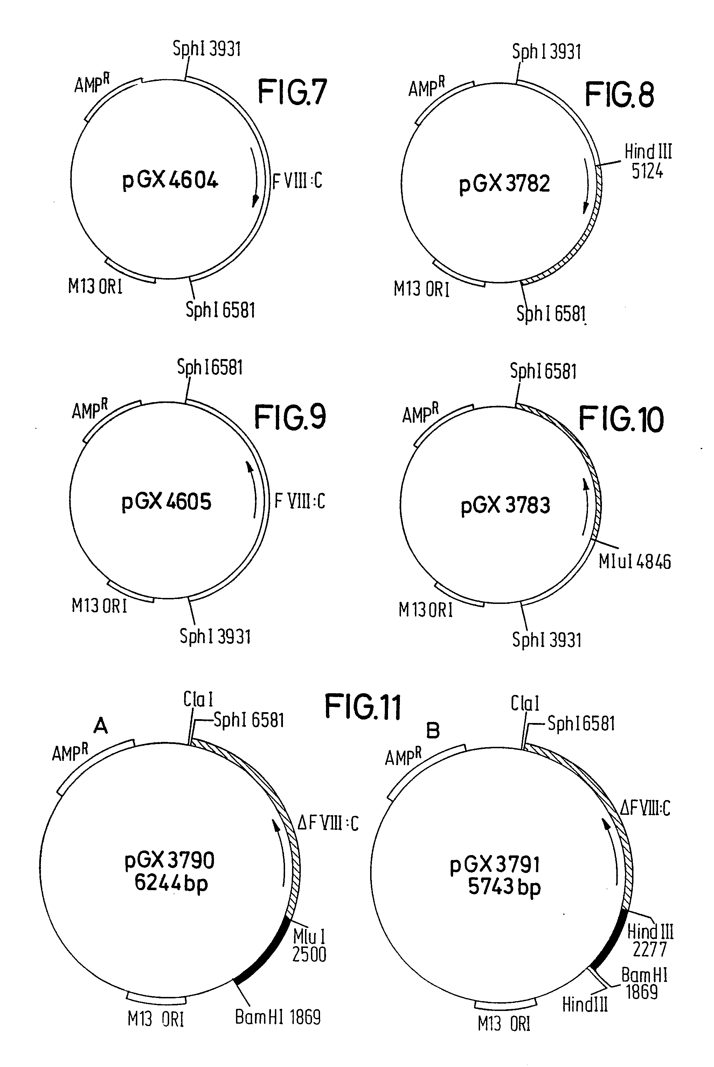

Der nichtkodierende Strang des Plasmids pGX4602 (Fig. 5) wurde isoliert und mit Hilfe des synthetischen Oligonukleotides 5'-AAA TAT GAG ACG CGT TCT GAT GAT - 3' eine neue MluI-Stelle (Position 2500) an der Codon-Position 814/815 (ACT TTT → ACG CGT) in die F VIII:C-Sequenz eingeführt. Das resultierende Plasmid ist pGX3787 (Fig. 6).
Der nichtkodierende Strang des Plasmids pGX4604 (Fig. 7) wurde, wie oben beschrieben, isoliert und mit Hilfe des synthetischen Oligonukleotides 5'-AAT CAG AGC CCC CGA AGC TTT CAA AAG-3' eine neue HindIII-Stelle (Position 5124) an den Codon-Positionen 1689/1691 (CGC AGC TTT → CGA AGC TTT) in die F VIII:C-Sequenz eingeführt. Das resultierende Plasmid ist pGX3782 (Fig. 8).
Der codierende Strang des Plasmids pGX4605 (Fig. 9) wurde ebenfalls isoliert und als Template DNA zusammen mit dem synthetischen Oligonukleotid 5'-CAG GGA CAA ACG CGT ATC CTT TTT CTT-3' dazu verwendet, eine neue MluI-Stelle (Position 4846) an der Codon-Position 1597/1598 (ACC ATT → ACG CGT) in die F VIII:C-Sequenz einzuführen. Das resultierende Plasmid ist pGX3783 (Fig. 10).

## d) Zusammenbau der Plasmide pGX3788 und pGX3789

Im folgenden wurden die Plasmide pGX3788 (= F VIII: CΔ741-1689) und pGX3789 (= F VIII:CΔ816-1598) durch Hinzufügen der fehlenden 5′ und 3′ F VIII:C-Regionen hergestellt. Der Zusammenbau erfolgte in mehreren Schritten.

## e) pGX3788

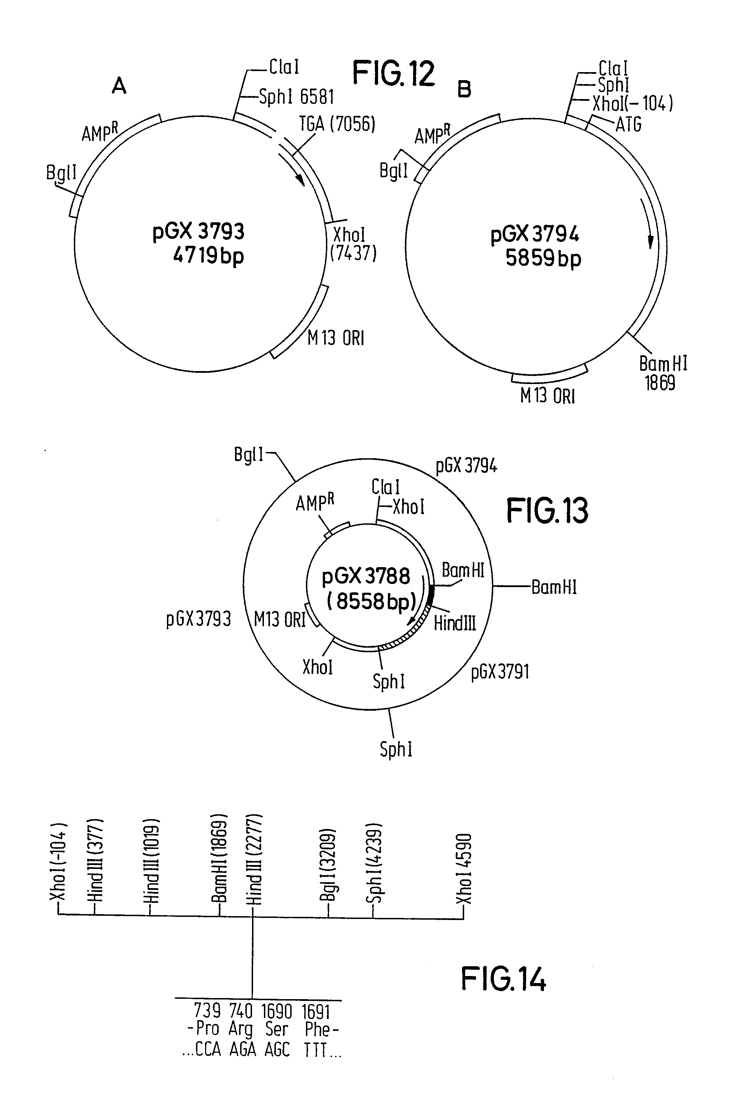

Das isolierte HindIII (Position 5124)-SphI (Position 6581) Fragment von pGX 3782 (Fig. 8, schraffierter Teil) wurde mit dem isolierten BamHI (Position 1869)-HindIII (Position 2277) Fragment von pGX4602 (Fig. 5) und mit dem mit alkalischer Phosphatase behandelten großen BamHI-SphI-Fragment von pGX2683 ligiert. Das Gemisch wurde in den E. coli Stamm JM101 transformiert und Plasmid-DNA von Ampicillin-resistenten Kolonien restriktionsendonukleolytisch analysiert. Plasmid-DNA eines korrekten Klones, pGX3791, wurde isoliert und die Deletion durch Sequenzierung bestätigt (Fig. 11B).

pGX3788 wurde durch Ligierung der in Fig. 13 gezeigten Fragmente konstruiert. pGX3794 trägt das 5′-Ende der F VIII: C-Sequenz als XhoI (Position-104)-BamHI (Position 1869) Fragment (Fig. 12B) und pGX3793 trägt das 3′-Ende der F VIII: C-Sequenz als SphI (Position 6581)-XhoI (Position 7437) Fragment (Fig. 12A). Die Fragmente wurden so miteinander ligiert, daß der Translationsleserahmen von F VIII:C beibehalten ist und die Codone 740 (Arg) und 1960 (Ser) mittels einer HindIII-Stelle fusioniert sind (Fig. 14). Somit trägt das durch pGX3788 spezifizierte Molekül eine Deletion, die die Aminosäuren 741-1689 des Faktor VIII:C umfaßt.

## f) pGX3789

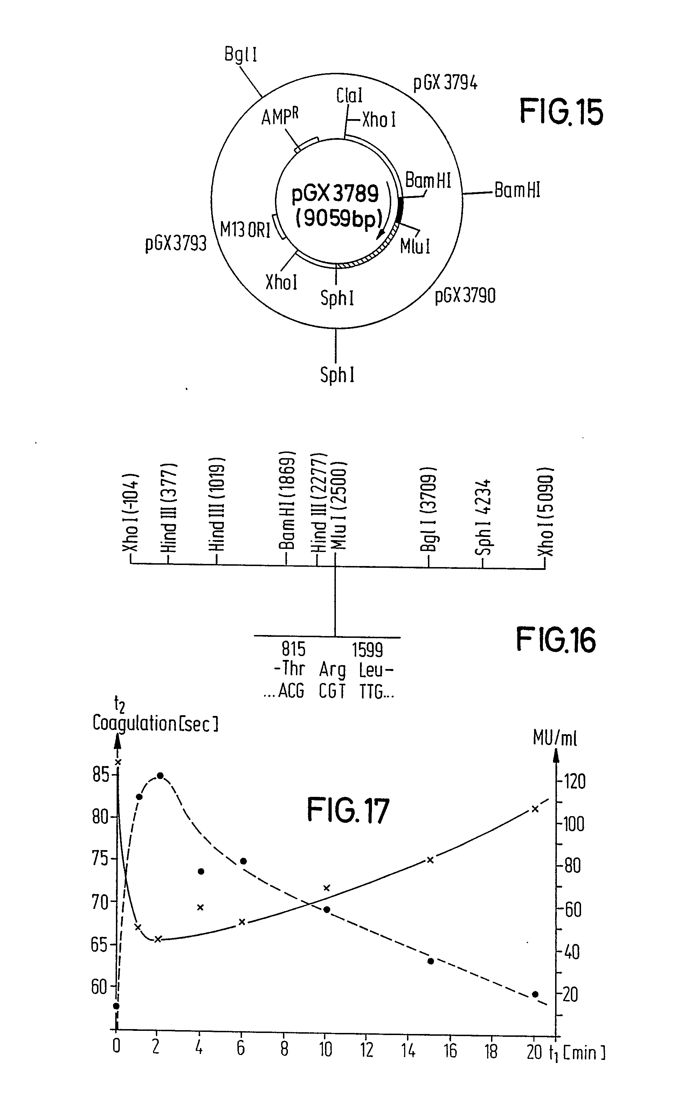

Zunächst wurde Plasmid pGX3790 (Fig. 11A) durch Ligieren folgender Fragmente konstruiert: MluI (Position 4846)-SphI (Position 6581) Fragment von pGX3783, BamHI (Position 1869)-MluI (Position 2500) Fragment von pGX3787 und das große, mit alkalischer Phosphatase behandelte BamHI-SphI-Fragment von pGX2683. Die Plasmid DNA des nach Restriktionsanalyse korrekten Klones, pGX3790, wurde isoliert und die Deletion durch Sequenzieren bestätigt (Fig. 11A). Anschließend wurde pGX3789 durch Ligieren der in Fig. 15 gezeigten Fragmente konstruiert. Die Fragmente wurden so miteinander ligiert, daß der Translationsleserahmen von F VIII:C beibehalten wird und die Codone 815 (Thr) und 1599 (Leu) mittels einer MluI-Stelle fusioniert wurden, wobei zwischen 815 (Thr) und 1599 (Leu) eine zusätzliche Aminosäure (Arginin) hinzugefügt wird (Fig. 16). Somit trägt das durch pGX3789 spezifizierte Molekül eine Deletion, die die Aminosäuren 816-1598 des F VIII:C umfaßt. Fig. 3 zeigt die Restriktionskarte von pGX3254 im Vergleich zu den Deletionsmutanten pGX3788 und pGX3789.

## 3. Konstruktion von F VIII:C Expressionsplasmiden

Das 7540 Basenpaar große XhoI Fragment aus Plasmid pGX3254, welches die gesamte F VIII:C Kodierungssequenz trägt, wurde isoliert, mit Hilfe des Klenow-Fragmentes der DNA-Polymerase aufgefüllt und in die aufgefüllte XbaI-Stelle des Vektors pZET4 bzw. in die aufgefüllte XbaI-Stelle des Vektors pSVASTOP 1 ligiert. Der Vektor pZET4 benutzt den SV40 Promotor zur Expression heterologer Gene und besitzt mRNA Splice-Signale (Offenlegungsschrift DE 36 21 371 A1). Der Vektor pSVASTOP1 benutzt ebenfalls den SV40-Promotor, trägt aber keine mRNA Splice-Signale (Offenlegungsschrift DE 36 24 453 A1). Die resultierenden Plasmide, welche das F VIII:C Fragment in der korrekten Orientierung relativ zum SV40 Promotor von pZET4 bzw. von pSVASTOP1 tragen, sind pZF VIII:C, bzw. pSV F VIII:C.

Das 4694 Basenpaar große XhoI Fragment aus pGX3788, welches die Kodierungsregion der Deletionsmutante F VIII:CΔ741-1689 trägt, wurde isoliert, aufgefüllt und in die aufgefüllte XbaI-Stelle des Vektors pZET4 bzw. in die aufgefüllte XbaI-Stelle des Vektors pSVASTOP1 ligiert. Die resultierenden Plasmide, welche das F VIII:C Fragment in der korrekten Orientierung relativ zum SV40 Promoter in pZET4 bzw. pSVASTOP1 tragen, sind pZF VIII:CΔ741-1689 bzw. pSVF VIII:CΔ741-1689.

Das 5195 Basenpaar große XhoI Fragment aus pGX3789, welches die Kodierungsregion der Deletionsmutante F VIII:CΔ816-1598 trägt, wurde isoliert, aufgefüllt und in die aufgefüllte Xbal-Stelle des Vektors pZET4 bzw. in die aufgefüllte Xbal-Stelle des Vektors pSVASTOP1 ligiert. Die resultierenden Plasmide, welche das F VIII:C Fragment in der korrekten Orientierung relativ zum SV40 Promotor in pZET4 bzw. in pSVASTOP1 tragen, sind pZF VIII:CΔ816-1598 bzw. pSVF VIII:CΔ816-1598.

### 4. Expression von Faktor VIII:C in animalen Zellen

Die in Abschnitt 3 beschriebenen Faktor VIII:C Konstruktionen wurden mit zwei verschiedenen DHFR-Plasmiden (pSVOAdhfr, Offenlegungsschrift DE 36 24 453 A1, oder pSV2dhfr, Lee et al., Nature 294, (1981) 228-232) in DHFR⁻ CHO-Zellen (Urlaub und Chasin (1980), Proc. Natl. Acad. Sci., USA 77, 4216-4220), oder mit dem Vektor pRMH140 (Hudziak et al., Cell 31 (1981), 137-146) in BHK-Zellen kotransfiziert. Anschließend wurden die transfizierten Zellen auf den DHFR⁺ NEO⁺ Phänotyp selektioniert. Die Überstände der durch Selektion erhaltenen Zellklone wurden auf die Produktion von F VIII:C hin untersucht.

Der Nachweis erfolgte mit drei verschiedenen Methoden:

i) Immunologischer Nachweis über ELISA. Hierbei wurde die Kombination aus polyklonalem Antiserum gegen F VIII:C (z.B. K F VIII:C Cag: 9460-010886; Behringwerke) und den monoklonalen Antikörpern ESH2, ESH8 (Bioscott) konjugiert mit Peroxidase verwendet. Die untere Detektionsgrenze lag bei 1-3 ng F VIII:C/ml.

ii) Biologischer Aktivitätstest mit dem COA-Test F VIII:C (Kabi Vitrum). Die untere Detektionsgrenze lag bei 1-3 ng F VIII:C/ml.

iii) Coagulationstest nach Lee et al., Throm. Res. 30 (1983) 511-519.

### a) Expression in CHO-Zellen:

Die F VIII:C Plasmide pSV F VIII:C, pSV F VIII:CΔ816-1598, pSV F VIII:CΔ741-1689, pZ F VIII:C, pZ F VIII:CΔ816-1598 oder pZ F VIII:CΔ741-1689 wurden jeweils mit einem der DHFR-Vektoren pSVOAdhfr oder pSV2dhfr mittels der Calciumphosphatkopräzipitationsmethode (Graham und van der Eb, Virology 52 (1973), 456-467) in CHO-Zellen kotransfiziert.

Hierzu wurden jeweils 20 μg der F VII:C Expressionsplasmide mit 5 μg der DHFR Vektoren (pSV2dhfr bzw. pSVOAdhfr) gemischt, kopräzipitiert und anschließend zur Transfektion (0,5-1 x 10⁶ Zellen in einer 25 cm² Kulturschale) eingesetzt. Nach 3 Tagen wurden die Zellen trypsiniert, in 60 mm Petrischalen übertrgen und mit Selektionsmedium (ohne Glycin, Hypoxanthin und Thymidin) versetzt. Unter diesen Bedingungen überleben nur Zellen, die stabil mit dem DHFR-Gen transfiziert worden sind.

Nach 1-3 Wochen wurden einzelne Zellklone isoliert und in Medium ohne Glycin, Hypoxanthin und Thymidin vermehrt. Die Überstände von insgesamt 73 Zellklonen wurden getestet, wobei in 12 Überständen F VIII:C sowohl immunologisch als Antigen als auch über biologische Aktivität nachgewiesen werden konnte. Tab. 2 repräsentiert eine Übersicht über die Verteilung der F VIII:C exprimierten Zellklone bezogen auf die transfizierten Konstrukte in CHO-Zellen.

Als Basisexpression vor Amplifikation mit Methotrexat wurden mit Hilfe des ELISA-Nachweises 2-3 ng/ml an F VIII:C Antigen gefunden. Die biologische Aktivität der F VIII:C exprimierten Klone lag zwischen 8 und 14 MU/ml/10⁶ Zellen.

### b) Expression von F VIII:C in BHK-Zellen

Je 20 μg der F VIII:C-Expressionsplasmide pSV F VIII:C, pSV F VIII:CΔ816-1598, pSV F VIII:CΔ741-1689, pZ F VIII:C, pZ F VIII:CΔ816-1598 oder pZ F VIII:CΔ741-1689 wurden mit 10 μg des für G418-Resistenz kodierenden Plasmids pRMH140 nach der in a) beschriebenen Calciumphosphatkopräzipitationsmethode in BHK-Zellen kotransfiziert. Nach 3 Tagen wurden die Zellen trypsiniert, in drei 60 mm Petrischalen übertragen und mit Selectionsmedium, das 400 μg/ml G418 enthielt, versetzt. Nach 12-14 Tagen wurden G418 resistente Zellklone isoliert und vermehrt. Insgesamt wurden die Überstände von 45 Zellklonen auf Antigen und biologische Aktivität hin mit den in a) beschriebenen Methoden untersucht, wobei 19 Überstände positiv in Bezug auf F VIII:C reagierten. Tab. 3 gibt eine Übersicht über die Verteilund der F VIII:C exprimierenden Zellklone bezogen auf die transfizierten Konstrukte in BHK-Zellen.

Die durchschnittliche biologische Aktivität der F VIII:C exprimierenden Zellklone lag bei 12 MU/ml/10⁶ Zellen. Im ELISA konnten 2-4 ng/ml an F VIII:C Antigen nachgewiesen werden.

Der Kulturüberstand eines neuen Zellklones, welcher mit dem Konstrukt pZ F VIII:CΔ741-1689 transfiziert worden war, wurde in Bezug auf die Aktivierbarkeit der exprimierten Deletionsmutante mit Thrombin hin untersucht (Toole et al., Nature 312 (1984), 342-347). Hierzu wurde der Kulturüberstand mit 1 U/ml Thrombin versetzt und bei Raumtemperatur inkubiert. Zu verschiedenen Zeiten ($t_1$) wurden Aliquots entnommen und die biologische Aktivität mit dem Coagulationstest bestimmt. Das Ergebnis dieses Experimentes ist in Fig. 17 wiedergegeben. Dabei sind die Coagulationszeiten ($t_2$) mit (-x-) gekennzeichnet und die entsprechenden MU/ml mit (-•-) angegeben, wobei der Einfluß von α-Thrombin (1 U/ml) auf die Aktivität des F VIII:CΔ741-1689 Moleküls gemessen wird. Aus dem Versuch geht hervor, daß das F VIII:CΔ741-1689 Molekül durch den Einfluß von Thrombin etwa um den Faktor 10 aktiviert wird.

Weiterhin wurde im Vergleich mit der Mutante F VIII:CΔ816-1598 und mit natürlichen F VIII:C gezeigt, daß die Mutante F VIII:CΔ816-1598 eine wesentlich längere Inaktivierungszeit besitzt.

**Tabelle 1:**

**F VIII:C-spezifische Oligonukleotide**

| Oligonukleotid Nr. | Position[*] |
|---|---|
| **kodierender Strang:** | |
| 1572 | 1 - 20 |
| 1609 | 301 - 317 |
| 1610 | 962 - 978 |
| 1613 | 1761 - 1777 |
| 1611 | 3429 - 3445 |
| 1573 | 4141 - 4160 |
| 1612 | 4992 - 5008 |
| 1614 | 6141 - 6157 |
| **nicht-kodierender Strang:** | |
| 1492 | 7198 - 7179 |
| 1493 | 6465 - 6446 |
| 1494 | 5685 - 5666 |
| 1495 | 5092 - 5073 |
| 1531 | 2400 - 2351 |
| 1496 | 2339 - 2320 |
| 1497 | 1179 - 1160 |
| 1498 | 570 - 551 |
| 1499 | 120 - 101 |

[*] Position in bp bezogen auf die Sequenz von Wood et al. (1984) Nature 312, 330-337

Tabelle 2: Faktor VIII:C-exprimierende CHO-Klone

A) Kotransfiziert mit pSV2dhfr:

| Plasmid | Anzahl DHFR$^+$-Klone | Positiv in Bezug auf F VIII:C Expression (ELISA; COA-Test) |
|---|---|---|
| pSV F VIII:C | 6 | 0 |
| pSV F VIII:CΔ816-1598 | 8 | 1 |
| pSV F VIII:CΔ741-1689 | 0 | 0 |
| pZ F VIII:C | 2 | 0 |
| pZ F VIII;CΔ816-1598 | 13 | 5 |
| pZ F VIII:CΔ741-1689 | 4 | 0 |

B) Kotransfiziert mit pSVOAdhfr:

| Plasmid | Anzahl DHFR$^+$-Klone | Positiv in Bezug auf F VIII:C Expression |
|---|---|---|
| pSV F VIII:C | 14 | 2 |
| pSV F VIII:CΔ816-1598 | 6 | 0 |
| pSV F VIII:CΔ741-1689 | 6 | 1 |
| pZ F VIII:C | 4 | 1 |
| pZ F VIII:CΔ816-1598 | 7 | 2 |
| pZ F VIII:CΔ741-1689 | 3 | 0 |
| | 73 | 12 |

## Tabelle 3: Faktor VIII:C-exprimierende BHK-Klone
### kotransfiziert mit pRMH140

| Plasmid | Anzahl Neo$^+$-Klone | Positiv in Bezug auf F VIII:C-Expression (ELISA; COA-Test) |
|---|---|---|
| pSV F VIII:C | 7 | 1 |
| pZ F VIII:CΔ741-1689 | 22 | 9 |
| pZ F VIII:CΔ816-1598 | 9 | 3 |
| pSV F VIII:CΔ816-1598 | 7 | 6 |
| | 45 | 19 |

**Ansprüche**

1. Faktor VIII:C-Derivat, dadurch gekennzeichnet, daß die natürliche Thrombin-Spaltstelle bei Arg$^{372}$ erhalten bleibt, eine neue bei Arg$^{740}$ entstanden ist und die vorhandenen bei Arg$^{1648}$ und Arg$^{1689}$ fehlen.

2. Faktor VIII:C-Derivat nach Anspruch 1, dadurch gekennzeichnet, daß die Aminosäuren 741 bis 1689 des authentischen Moleküls deletiert sind.

3. DNA-Sequenzen, die für die in Anspruch 1 oder 2 genannten Faktor VIII:C-Derivate kodieren, sowie Allele und Varianten.

4. Arzneimittel, gekennzeichnet durch einen Gehalt an einem Faktor VIII:C-Derivat nach Anspruch 1 oder 2.

5. Injektionslösung, gekennzeichnet durch einen Gehalt an einem Arzneimittel nach Anspruch 4.

6. Verwenduing der Faktor VIII:C-Derivate nach Anspruch 1 oder 2 zur Herstellung von Arzneimitteln für die Therapie von Gerinnungsstörungen.

7. DNA-Strukturen und Vektoren, die DNA-Sequenzen nach Anspruch 3 enthalten.

8. Pro- oder eukaryotische Zellen, DNA-Strukturen oder Vektoren nach Anspruch 7 enthalten.

9. Verfahren zur Herstellung von Faktor VIII:C-Derivaten, dadurch gekennzeichnet, daß Zellen nach Anspruch 8 zur Expression gebracht werden.

10. Verwendung von Faktor VIII:C-Derivaten nach Anspruch 1 oder 2 zur Herstellung von Diagnostika.

Patentansprüche für folgende Vertragsstaaten : GR, ES

1. Verfahren zur Herstellung von Faktor VIII:C-Derivaten, dadurch gekennzeichnet, daß die natürliche Thrombinspaltstelle bei Arg$^{372}$ erhalten bleibt, eine neue bei Arg$^{740}$ entsteht und die vorhandenen bei Arg$^{1648}$ und Arg$^{1689}$ verschwinden.

2. Verfahren zur Herstellung von Faktor VIII:C-Derivaten nach Anspruch 1, dadurch gekennzeichnet, daß die Aminosäuren 741 bis 1689 des authentischen Moleküls deletiert sind.

3. Verfahren zur Herstellung von Faktor VIII:C-Derivaten nach Anspruch 1, dadurch gekennzeichnet, daß die für die Faktor VIII:C-Derivate kodierende DNA in Bakterien, Hefen, animalen oder humanen Zellen exprimiert wird.

4. Verfahren zur Herstellung eines Arzneimittels, dadurch gekennzeichnet, daß ein nach Anspruch 1, 2 oder 3 hergestelltes Faktor VIII: C-Derivat mit Trägersubstanzen und/oder Puffer gemischt wird.

FIG.1

FIG.2

FIG.3

(a) pGX 3254

- XhoI
- ATG
- EcoRI
- HindIII (377)
- EcoRI (561)

1
- HindIII (1019)
- AccI (1033)
- PvuII (1038)
- BglII (1680)
- BamHI (1869)  PvuII 1850

2
- HindIII (2277)
- EcoRI (2289)
- SauI (2765)
- SauI (2987)

3
- SauI (3449)
- ScaI (3795)
- SphI (3931)

4
- BglII (4210)
- BamHI (4743)

5
- BglI (6056)

6
- SphI (6581)
- SauI (6718)
- XbaI (6945)   EcoRI (6977)

7
- SauI (7011)   TGA (7054)
- PvuII (7267)
- Xho (7437)

- XhoI (−104)
- XbaI (−94)
- EcoRI (−90)
- EcoRI (−79)
- −71
- ATG (1)
- SacI (10)
- EcoRI (214)

(b) pGX 3788
- HindIII (2277)
- HindIII (5124)

(c) pGX 3789
- MluI (2500)
- MluI (4846)

FIG.4

FIG.5

FIG.6

FIG.7 — pGX4604: SphI 3931, AMP^R, F VIII:C, M13 ORI, SphI 6581

FIG.8 — pGX3782: SphI 3931, AMP^R, Hind III 5124, M13 ORI, SphI 6581

FIG.9 — pGX4605: SphI 6581, AMP^R, F VIII:C, M13 ORI, SphI 3931

FIG.10 — pGX3783: SphI 6581, AMP^R, MIuI 4846, M13 ORI, SphI 3931

FIG.11 — A pGX3790 6244bp: ClaI, SphI 6581, AMP^R, ΔF VIII:C, MIu I 2500, BamHI 1869, M13 ORI; B pGX3791 5743bp: ClaI, SphI 6581, AMP^R, ΔF VIII:C, Hind III 2277, BamHI 1869, HindIII, M13 ORI

FIG.12

A

ClaI
SphI 6581
TGA (7056)
AMP^R
BglI
pGX 3793
4719 bp
XhoI
(7437)
M13 ORI

B

ClaI
SphI
XhoI(−104)
ATG
AMP^R
BglI
pGX 3794
5859 bp
BamHI
1869
M13 ORI

FIG.13

BglI
AMP^R
ClaI
XhoI
pGX 3794
pGX 3788
(8558 bp)
BamHI
BamHI
HindIII
pGX 3793
M13 ORI
XhoI
SphI
pGX 3791
SphI

FIG.14

XhoI(−104)
HindIII (377)
HindIII (1019)
BamHI (1869)
HindIII (2277)
BglI (3209)
SphI (4239)
XhoI 4590

739  740  1690  1691
-Pro  Arg  Ser  Phe-
...CCA AGA AGC  TTT...

FIG.15

FIG.16

FIG.17